# EUROPEAN PATENT APPLICATION

(11) **EP 4 591 859 A1**
(43) Date of publication of application: **30.07.2025**
(21) Application number: 23876398.1
(22) Date of filing: 04.09.2023
(51) Int. Cl.: A61K 9/51, A61K 48/00, A61K 31/203, A61P 35/00, A61P 31/00, A61K 39/00

(54) **LIPID NANOPARTICLE, PREPARATION METHOD THEREFOR, AND USE THEREOF**

(30) Priority: 11.10.2022 CN 202211243350
(71) Applicant: INSTITUTE OF CHEMISTRY, CHINESE ACADEMY OF SCIENCES, Beijing 100190 (CN); Proxybio Therapeutics Co., Ltd., Shenzhen, Guangdong 518000 (CN)
(72) Inventor: LV, Xueguang, Beijing 100190 (CN); LIN, Jiaqi, Shenzhen, Guangdong 518000 (CN); LI, Wei, Beijing 100190 (CN); LI, Jingjiao, Beijing 100190 (CN); CHENG, Xingdi, Beijing 100190 (CN)
(74) Representative: Bayramoglu et al.
(86) International application number: PCT/CN2023/116854
(87) International publication number: WO 2024/078193

(57) **Abstract**

The invention discloses a lipid nanoparticle and a preparation method and application thereof are provided. The lipid nanoparticle (LNP) includes a carrier and an encapsulated nucleic acid, the carrier includes an ionizable lipid, a helper phospholipid, a PEGylated lipid, cholesterol and its derivatives, and a retinoid compound; and the nucleic acid is one or more of mRNA, circRNA, siRNA, microRNA, antisense nucleic acid, and plasmid. The invention proves that the five-component LNP can activate the triple immune response of body fluid, cells and mucosa after intramuscular or subcutaneous administration, and has important application prospects in the field of infectious disease vaccines and mucosal-related tumor vaccines.

## Description

This application requires the applicant to submit the priority of the patent with an application number of 202211243350.1 and the invention name of 'lipid nanoparticle and preparation method and application thereof to the National Intellectual Property Office of China on October 11, 2022. The full text of the earlier application is incorporated in this application by reference.

### TECHNICAL FIELD

The invention relates to the field of pharmaceutical technology, specifically, a five-component lipid nanoparticle (LNP) that can encapsulate both nucleic acid drugs/vaccines and retinoid compounds simultaneously, a preparation method and an application thereof.

### BACKGROUND TECHNOLOGY

After the outbreak of the COVID-19 pandemic, the U.S. Food and Drug Administration approved messenger RNA (mRNA) COVID-19 vaccines from two companies, Moderna and Pfizer-BioNTech, in record time, giving mRNA vaccines unprecedented attention. Compared with traditional protein, virus, and DNA vaccines, the mRNA vaccines have the advantages of encoding a plurality of proteins, not needing to enter the nucleus of cells, and rapidly producing in large quantities. However, due to the large molecular weight and negative charge of mRNA, it cannot enter the cell autonomously, and mRNA is easily degraded by enzymes in the body, resulting in poor stability. Therefore, the key to the development of mRNA vaccines is to develop drug delivery systems that can improve their entry efficiency and stability into cells.

Lipid nanoparticles (LNP) are currently the most widely used mRNA delivery system in clinical practice. The LNP currently approved for clinical delivery of the COVID-19 mRNA vaccine consists of four components: ionizable lipids, helper phospholipids, PEGylated lipids, and cholesterol. After intramuscular injection, the mRNA vaccines can activate both humoral and cellular immune responses and produce high levels of neutralizing antibodies and killer T cells to effectively kill viruses invading the body. However, the COVID-19 mRNA vaccine based on LNP carrier still faces the disadvantages of strong side effects and inability to activate mucosal immune responses. The vast majority of infectious diseases, including the SARS-CoV-2, are transmitted through the respiratory tract, the use of mRNA vaccines to activate mucosal immune responses can cut off the transmission route of pathogens from the source, which is expected to greatly shorten the transmission time of pathogens and effectively control the spread of the epidemic. Therefore, there is still a strong demand for the development of new mRNA delivery carriers that can activate the mucosal immune response more effectively.

### CONTENT OF THE INVENTION

In order to improve the above technical problems, the invention provides a novel LNP carrier that can simultaneously encapsulate nucleic acid drugs (mRNA, siRNA, microRNA, antisense nucleic acid, etc.) and retinoid compounds, and a preparation method and an application. Five-component LNP is formed by adding a certain proportion of retinoid compounds to the four-component LNP used in clinical practice, the addition of retinoid compounds not only significantly improves the cell entry ability and mRNA expression efficiency of the original carrier, but also induces the immune cells activated by the vaccine to homing to the mucosa and activate the mucosal immune response. The liposome carrier also improves the water solubility of retinoid compounds and solves the shortcomings of dependency of retinoid compounds on polyethylene glycol or oily solvent delivery. The invention proves that the five-component LNP can activate the triple immune response of body fluids, cells and mucosa after intramuscular or subcutaneous administration, and has important application prospects in the field of infectious disease vaccines and mucosal-related tumor vaccines.

The invention provides a lipid nanoparticle (hereinafter referred to as LNP), including a carrier and an encapsulated nucleic acid, the carrier includes an ionizable lipid, a helper phospholipid, a PEGylated lipid, cholesterol and derivatives thereof, and a retinoid compound; and the nucleic acid is at least one of mRNA, circRNA, siRNA, microRNA, antisense nucleic acid, and plasmid.

According to the embodiment of the invention, the ionizable lipid accounts for 20 mol %-50 mol % of total lipids in the LNP, for example, 25 mol %, 30 mol %, 35 mol %, 40 mol %, 45 mol %.

According to the embodiment of the invention, the ionizable lipid is selected from at least one of the following compounds, including but not limited to: 8-[(2-hydroxyethyl)(6-oxo-6-decyloxyhexyl)amino]octanoic acid(heptadecan-9-yl)ester(SM-102), [(4-hydroxybutyl)azetidinyl]bis(hexane-6,1-diyl)bis(2-hexyldecanoate)(ALC-0315), 4-(N,N-dimethylamino)butanoic acid(dioleyl)methyl ester(DLin-MC3-DMA), 3,6-bis{4-[bis(2-hydroxydodecyl)amino]butyl}piperazine-2,5-dione(cKK-E12), 9-(4-(dimethylamino)butyryloxy)heptadecanedioic acid di((Z)-nonly-2-en-1-yl)ester(L319), N2,2-dimethylene-4-(dimethylamino)ethyl[1,3]-dioxolane(DLin-KC2-DMA), 8-[(2-hydroxyethyl)(8-nonyloxy-8-oxooctyl)amino]octanoic acid(heptadecan-9-yl)ester (Lipid5), 1,1'-[(2-{4-[2-({2-[Bis(2-hydroxydodecyl)amino]ethyl}(2-hydroxydodecyl)amino)ethyl ]piperazin-1-yl}ethyl)azabicyclo[bis(dodecane)-2-alcohol)(C12-200), (2,3-dioleoyl-propyl)trimethylammonium chloride(DOTAP), dimethyldioctadecylammonium bromide (DDAB) and tetrakis(8-methylnonyl)3,3',3",3"'-{[(methylazanediyl)bis(propane-3,1diyl)]bis(azatriyl) }tetrapropionate(306Oi10); preferably, 8-[(2-hydroxyethyl)(6-oxo-6-decyloxyhexyl)amino]octanoic acid(heptadecan-9-yl)ester(SM-102) and/or [(4-hydroxybutyl)azetidinyl]bis(hexane-6,1-diyl)bis(2-hexyldecanoate)(ALC-0315).

According to the embodiment of the invention, the helper phospholipid accounts for 2 mol %-10 mol % of the total lipids in the LNP, for example, 3 mol %, 4 mol %, 5 mol %, 6 mol %, 7 mol %, 8 mol %, 9 mol %.

According to the embodiment of the invention, the helper phospholipid is selected from at least one of the following compounds, including but not limited to: 1,2-distearoyl-sn-glycero-3-phosphatidylcholine (DSPC), 1,2-dioleoyl-sn-glycero-3-phosphatidylcholine (DOPC), 1,2-dipalmitoyl-sn-glycero-3-phosphatidylcholine (DPPC), 2-oleoyl-1- palmitoyl acyl-sn-glycero-3-phosphatidylcholine (POPC), 1,2-dioleoyl-sn-glycero-3-phosphatidylethanolamine (DOPE), 2-oleoyl-1-palmitoyl-sn-glycero-3-phosphatidylethanolamine (POPE), 1,2-distearoyl-sn-glycero-3-phosphatidylethanolamine (DSPE), and 1,2-dipalmitoyl-sn-glycero-3-phosphatidylethanolamine (DPPE), preferably, DSPC.

According to the embodiment of the invention, the cholesterol and derivative thereof account for 10 mol %-40 mol % of the total lipids in the LNP, for example, 15 mol %, 20 mol %, 25 mol %, 30 mol %, 35 mol %.

According to the embodiment of the invention, the cholesterol and derivative thereof are selected from at least one of the following compounds, including but not limited to: β-sitosterol, cholestanol, cholestanone, cholesterol, cholestenone, 7β-hydroxycholesterol, and 7α-hydroxycholesterol, preferably, cholesterol.

According to the embodiment of the invention, the PEGylated lipid accounts for 0.3 mol % -30 mol % of the total lipids in the LNP, preferably, 0.5 mol % -2.5 mol %, for example, 1.0 mol %, 1.5 mol %, 2.0 mol %.

According to the embodiment of the invention, the PEGylated lipid is selected from at least one of the following compounds, including but not limited to: 1,2-dimyristoyl-rac-glycero-3-methoxypolyethylene glycol (DMG-PEG), 1,2-distearoyl-rac-glycero-3-methoxypolyethylene glycol (DSG-PEG), 1,2-dipalmitoyl-rac-glycero-3-methoxy polyethylene glycol (DPG-PEG), and 1,2-distearoyl-sn-glycero-3-phosphatidylethanolamine-methoxypolyethylene glycol (DSPE-PEG), preferably, DMG-PEG.

According to the embodiment of the invention, the retinoid compound accounts for 1 mol %-35 mol % of the total lipids in the LNP, for example, 5 mol %, 10 mol %, 15 mol %, 20 mol %, 25 mol %, 30 mol %.

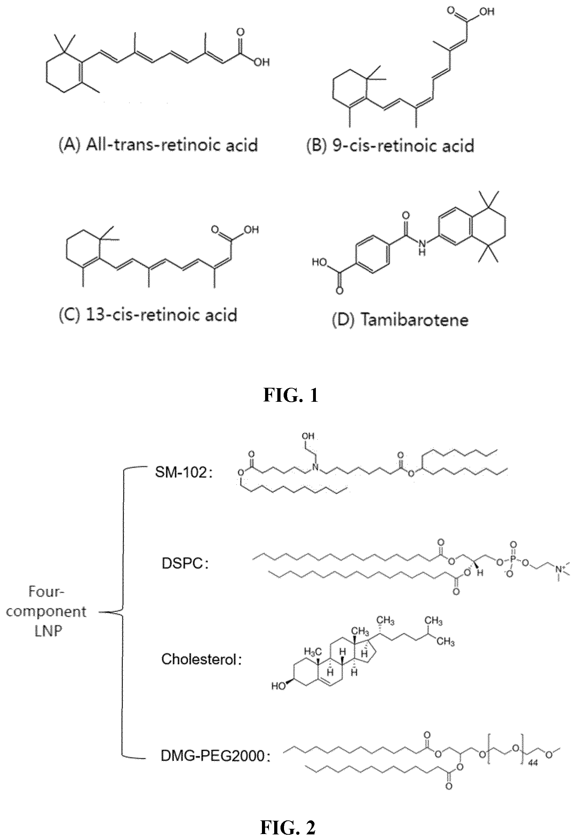

According to the embodiment of the invention, the retinoid compound is at least one of retinoic acid derivatives and synthetic retinoid compounds, for example, the retinoic acid derivatives include, but not limited to, at least one of all-trans-retinoic acid (ATRA), 9-cis-retinoic acid (9-cis-RA, 9-CRA), 13-cis-retinoic acid (13-cis-RA, 13-CRA), and the synthetic retinoid compounds include, but not limited to, retinoic acid receptor agonist such as tamibarotene (Am80). In some embodiments, the retinoid compound is preferably ATRA or Am80. The structural formulas of the compounds ATRA, 9-CRA, 13-CRA, and Am80 are shown in (A)-(D) of FIG. 1.

According to the embodiment of the invention, a molar ratio of the cholesterol and the derivatives thereof to the retinoid compound is within a range of 1:2-50:1, for example, 10:1-1:1, preferably, 4:1-1:1.

The 'total lipids' refer to a sum of the ionizable lipid, the helper phospholipid, the cholesterol and the derivatives thereof, the PEGylated lipid, and the retinoid compound.

According to the embodiment of the invention, the nucleic acids selected by the invention include but are not limited to at least one of the following nucleic acids: CpG-FAM, CpG-FAM, mRNA encoding firefly luciferase (mLuc), mRNA encoding the spike protein of the Omicron variant of SARS-CoV-2 virus (S-Omicron), mRNA encoding chicken ovalbumin (mOVA).

According to the embodiment of the invention, the encapsulation efficiency of the nucleic acid is 30-99 %, preferably, 70 %-90 %, for example, 70 %, 75 %, 80 %, 85 %, 90 %.

According to the embodiment of the invention, an encapsulation efficiency of the retinoid compound is 30-99 %, preferably 50-80 %, such as 50 %, 60 %, 65 %, 70 %, 80 %.

According to the embodiment of the invention, a molar ratio of nitrogen element of the ionizable lipid to phosphorus element of the nucleic acid is about (1-50):1, preferably, (5-20):1, for example, 5.67:1 or 15:1.

According to the embodiment of the invention, a hydrated particle size of the LNP is within a range of 100 nm-200 nm, for example, 120-180 nm.

In some embodiments, a molar ratio of the ionizable lipid, the helper phospholipid, the cholesterol, and the PEGylated lipid is 50:10:38.5:1.5.

In some embodiments, the carrier includes SM-102, DSPC, the cholesterol, DMG-PEG, and ATRA with a molar ratio of 36.1:7.2:27.8:1.1:27.8.

In some embodiments, the carrier includes SM-102, DSPC, the cholesterol, DMG-PEG, and AM80 with a molar ratio of 36.1:7.2:27.8:1.1:27.8.

In some embodiments, the LNP includes a carrier and an encapsulated nucleic acid,
the carrier is composed of the SM-102, the DSPC, the cholesterol, the DMG-PEG, and the ATRA with the molar ratio of 36.1:7.2:27.8:1.1:27.8, or the SM-102, the DSPC, the cholesterol, the DMG-PEG, and the AM80 with the molar ratio of 36.1:7.2:27.8:1.1:27.8; and the nucleic acid is CpG-FAM, mLuc, S-Omicron, or mOVA.

The invention also provides a preparation method of the LNP, including: obtaining the LNP based on an aqueous phase containing the nucleic acid and a lipid organic phase by an ethanol dilution method or a microfluidic method.

According to the embodiment of the invention, the ethanol dilution method includes the following steps: aspirating the aqueous phase containing the nucleic acid into the lipid organic phase, mixing a liquid by rapidly aspirating the liquid with a pipette greater than 50 times, standing after mixing to obtain the LNP.

According to the embodiment of the invention, the microfluidic method includes the following steps: aspirating the lipid organic phase and the aqueous phase containing the nucleic acid with a syringe, respectively, and injecting the lipid organic phase and the aqueous phase into a microfluidic chip at a flow rate of 1:3 for mixing, stopping collecting the liquid after the aqueous phase is empty in the syringe to obtain the LNP.

According to the embodiment of the invention, a volume ratio of the aqueous phase containing the nucleic acid to the lipid organic phase is 3: 1.

According to the embodiment of the invention, the ethanol dilution method further includes dialyzing the product in a 1×PBS buffer solution with a volume of greater than 1000 times of a volume of the product for more than 4 h.

According to the embodiment of the invention, the microfluidic method further includes dialyzing the collected mixture in a 1×PBS buffer solution with a volume of 1,000 times of a volume of the mixture for more than 4 h.

The invention also provides an application of the above LNP in a preparation of a biological product.

According to the embodiment of the invention, the biological product is an injectable biological product.

In some embodiments, the biological product is a vaccine, preferably, an mRNA vaccine.

In some embodiments, the biological product is selected from any of the following vaccines: an influenza vaccine, a human immunodeficiency virus (HIV) vaccine, a viral pneumonia (SARS, SARS-CoV-2, etc.) vaccine, a tuberculosis vaccine, a respiratory syncytial virus (RSV) vaccine, a rotavirus (RV) vaccine, a norovirus vaccine, an enterovirus (e.g., EV71) vaccine, an intestinal mucosal-related tumor vaccine (e.g., colorectal cancer mRNA vaccine), or a lung cancer vaccine, etc.

According to the embodiment of the invention, the administration method of the biological product is muscle administration or subcutaneous administration.

According to the embodiment of the invention, the biological product is used to prevent and/or treat mucosal transmission infectious diseases and mucosal-associated tumors. For example, the infectious disease is selected from influenza, novel coronavirus pneumonia (SARS-CoV-2), atypical pneumonia (SARS), tuberculosis, bronchitis and pneumonia due to RSV infection, AIDS, diseases caused by rotaviruses, diseases caused by noroviruses, and hand-foot-and-mouth disease due to enteroviral infections, for example, the mucosa-associated tumors can be colorectal tumors, lung cancer, etc.

According to the embodiment of the invention, the biological product is used for the treatment of in situ colorectal cancer.

The invention also provides a biological product having a meaning as described above.

The invention also provides a method for preventing and/or treating diseases, including applying the above biological products with effective amount of prevention and/or treatment to the subject;
the disease is a mucosa-transmitted infectious disease or a mucosal-associated tumor, for example, the disease is selected from any one of influenza, AIDS, viral pneumonia (SARS, SARS-CoV-2, etc.), tuberculosis, bronchitis and pneumonia due to RSV infection, diseases caused by rotaviruses, diseases caused by noroviruses, hand-foot-and-mouth disease due to enteroviral infections, colorectal tumors, lung cancer.

Beneficial effects:
(1) The invention provides a preparation method and application of a novel LNP carrier that can simultaneously encapsulate nucleic acid drugs (mRNA, circular RNA, siRNA, microRNA, antisense nucleic acid, plasmid) and retinoid compounds, the raw materials involved in this method are easy to obtain, the drug encapsulation rate is high, and the operation steps are simple.
(2) The five-component LNP containing the retinoid compound prepared by the invention can significantly improve the cell entry efficiency and mRNA expression efficiency of the original four-component LNP, and is universal in different LNP delivery carriers. The LNP carrier also solves the problem that retinoid compounds are insoluble in water and difficult to be injected clinically, and realizes the synergistic function of retinoid compounds and existing LNP carriers.
(3) The five-component LNP containing the retinoid compound prepared by the invention can simultaneously activate humoral, cellular, and mucosal immune responses after intramuscular or subcutaneous administration, it is expected to solve the problem that the existing four-component LNP mRNA carrier cannot activate the mucosal immune response, it has great application prospects in the field of prevention and treatment of infectious diseases transmitted through the mucosa.
(4) The five-component LNP containing the retinoid compound prepared in the invention can activate T cells in subcutaneous lymph nodes and induce their expression of intestinal homing factors after intramuscular or subcutaneous administration, improve the infiltration of antigen-specific tumor killer cells (CD8⁺T cells) in colorectal tumors, and effectively inhibit the growth of colorectal tumors in situ, it is expected to achieve a breakthrough in the field of colorectal tumor mRNA vaccine.

Definition and interpretation of terms

The term "a plurality of" means two, three, or more.

The term "effective amount" may refers to an amount of a biological product in the invention sufficient to achieve the intended application (including, but not limited to, treatment of the disease as defined above) determined according to methods available to a licensed clinician in this field. Determination of the effective dose for prevention and/or treatment is within the capabilities of a clinician or researcher in the field and may be altered by the intended application (in vitro or in vivo) or by the subject and disease condition being treated such as the subject's body weight and age, general health, severity of the disease condition, mode of administration, and other factors affecting efficacy such as history of drug allergy. The specific administered dose varies depending on the selected specific biological product, the dosing regimen relied upon, whether or not it is co-administered with other medications, the timing of the administration, the tissues in which the medication is administered, and the physical delivery system hosted.

The term "subject" in this invention refers to a specific human or other warm-blooded mammal. The subject may include adults and infants, children, and other warm-blooded mammals including, but not limited to, non-human primates such as chimpanzees, other apes or monkeys, and other zoo animals, domesticated mammals, or laboratory animals such as cats, pigs, dogs, cows, sheep, mice, rats, and guinea pigs. Preferably, the subject is preferably a human.

The term "Am80" and "AM80" refer to the same substance, i.e., tamibarotene.

### DESCRIPTION OF THE DRAWINGS

FIG. 1: A chemical structure of the retinoid compound used in Embodiment 1 of the invention.
FIG. 2: A composition and chemical structure of the commercial SM-102 four-component LNP.
FIG. 3: A hydrated particle size distribution curve and polydispersity index(PDI) of the sample obtained from Embodiment 1 of the invention.
FIG. 4: A hydrated particle size distribution curve and PDI of the sample obtained from Embodiment 2 of the invention.
FIG. 5: A quantitative high performance liquid chromatography (HPLC) spectrums of AM80 (A)(C) and ATRA (B)(D) and the corresponding peak area-concentration standard curve in Embodiment 3 of the invention.
FIG. 6: An average fluorescence intensity of DC2.4 cells detected by flow cytometry in Embodiment 4 of the invention. (Normalized by SM-102).
FIG. 7: Results of bioluminescence signal detected by the microplate reader in Embodiment 5 of the invention. (Normalized by SM-102).
FIG. 8: Results of bioluminescence signal detected by the microplate reader in Embodiment 6 of the invention. (Normalized by each control group).
FIG. 9: Results of bioluminescence signal detected by the microplate reader in Embodiment 6 of the invention. (Normalized by each control group).
FIG. 10: Results of bioluminescence signal detected by the microplate reader in Embodiment 7 of the invention. (Normalized by SM-102).
FIG. 11: (A) is a schematic diagram of the mechanism of flow cytometry detection of CCR9+T cells and α4β7+T cells in Embodiment 8 of the invention; (B) is a flow cytometry analysis result of the abilities of three experimental groups (PBS, free ATRA and mLuc@SM-102+ATRA) in activating CCR9+T cells and α4β7+T cells. (C) is a flow cytometry analysis result of the ability of different doses of Am80 and mLuc@SM-102+Am80 in activating CCR9+T cells and α4β7+T cells.
FIG. 12: In vivo imaging result of small animals in Embodiment 9 of the invention. (A) is a bioluminescence intensity of the injection site of three groups of mice (PBS, mLuc@SM-102 and mLuc@SM-102+ATRA); (B) is an in vitro imaging of mouse lymph nodes in (A); (C) is a bioluminescence intensity of the injection site of the three groups of mice(PBS, mLuc@SM-102 and mLuc@SM-102+AM80) and (D) is the luminescence signal intensity statistics.
FIG. 13: Flow chart of animal experiment in Embodiment 10 of the invention.
FIG. 14: Characterization results of humoral immune response of mice in the second week after enhanced immunization in Embodiment 10: (A) is a titer of specific IgG antibody against omicron S protein in the serum of mice, (B) is a titer of specific IgA antibody against omicron S protein in the serum of mice.
FIG. 15: Characterization results of cellular immune response of mice in the second week after enhanced immunization in Embodiment 10: (A) is a count of omicron S protein-specific CD4+T cells in the spleen of mice; (B) is a count of omicron S protein-specific CD8+T cells in the spleen of mice; (C) is a count of interferon type I (IFN-1)-secreting splenocytes after antigen stimulation in the spleen of mice.
FIG. 16: Characterization results of mucosal immune response of mice in the second week after enhanced immunization in Embodiment 10: (A) is a content of tissue-resident T cells in the lung tissue of mice; (B) is a titer of specific IgA antibody against omicron S protein in bronchoalveolar lavage fluid of mice; (C) is a titer of specific IgA antibody of omicron S protein in the feces of mice.
FIG. 17: Flow chart (A) of animal experiment in Embodiment 11 of the invention; characterization flow cytometry analysis (B) and quantitative result (C) of intratumoral antigen-specific killer T cell; in situ colorectal tumor photo (D) and tumor weight analysis (E) after treatment.
FIG. 18: Results of in situ tumor volume change and flow cytometry of mice on the 15^{th} day after immunization in Embodiment 11: (A) (B) are tumor volume and weight statistics; (C) is a content of killer T cells in tumor.
FIG. 19: Results of in vivo imaging of small animals of Embodiment 11 in the invention: (A) shows representative images of in vivo bioluminescence imaging of three groups of mice (PBS, mOVA@SM-102 and mOVA@SM-102+ATRA) at different periods; (B) is an average luminescence signal intensity value of the abdomen of the three groups of mice changed with time.
FIG. 20: Weight change curve of mice after receiving LNP immunization in Embodiment 11 of this invention.
FIG. 21: Case sections of the main organs of mice after receiving LNP immunization in Embodiment 11 and Embodiment 10 of this invention.
FIG. 22: Storage stability of LNP containing retinoid compounds in this invention.

### SPECIFIC IMPLEMENTATIONS

The above LNP can be prepared by the following methods: the ionizable lipid, phospholipid, PEGylated lipid, cholesterol and derivative thereof and a specific proportion of retinoid compounds are dissolved in ethanol to obtain the organic phase, and the nucleic acid molecules are dissolved in citrate buffer to obtain the aqueous phase. The aqueous phase and the organic phase are fully mixed, and LNP is prepared by ethanol dilution method or microfluidic method, and then dialyzed in PBS buffer solution to obtain LNP with a hydrated particle size of about 100-200 nm.

The LNP prepared by the invention has a uniform particle size, good dispersion, high encapsulation efficiency, good biocompatibility, and low toxicity and side effects, which can induce the production of intestinal homing factor by T cells and activate mucosal immune response.

The preparation method is as follows:

### (1) Preparation of LNP

The onizable lipids can be protonated under acidic conditions to form cationic lipids, which binds to negatively charged nucleic acid by electrostatic interaction to form the nucleic acid-containing LNP. In the LNP prepared in the invention, the nitrogen phosphorus molar ratio of nitrogen contained in the ionizable lipid to phosphorus contained in the encapsulated nucleic acid is 5.67:1, and the molar ratio of the ionizable lipid, the helper phospholipid, the cholesterol, and the PEGylated lipid is 50:10:38.5:1.5. The ionizable lipid is 8-[(2-hydroxyethyl)(6-oxo-6-decyloxyhexyl)amino]octanoic acid(heptadecan-9-yl)ester(SM-102); the helper phospholipid is 1,2-distearoyl-sn-glycero-3-phosphorylcholine(DSPC); and the PEGylated lipid is 1,2-dimyristoyl-sn-glycero-3-methoxypolyethylene glycol 2000(DMG-PEG2000). The components and structural formulas of the commercialized SM-102 four-component lipid are shown in FIG. 2.

For the preparation of the LNP with added retinoid compound, the five-component lipid is formed by adding a certain proportion of all-trans-retinoic acid (ATRA), 9-cis-retinoic acid (9-cis-RA or 9-CRA), 13-cis-retinoic acid (13-cis-RA or 13-CRA), or AM80, etc. each component is dissolved in the ethanol to form the organic phase.

CpG DNA modifying carboxy luciferin molecules (CpG-FAM) or mRNA encoding firefly luciferase protein (Firefly Luciferase mRNA, mLuc) is dissolved in 0.05 M citrate buffer to form the aqueous phase with a nucleic acid drug concentration of 0.02 mg/mL-0.17 mg/mL. The LNP is prepared by the microfluidic method or the ethanol dilution method, and then dialyzed in the 1 × PBS for more than 4 h.

Unless otherwise specified, the four-component LNP below includes the SM-102, the DSPC, the cholesterol, and the DMG-PEG2000 with the molar ratio of 50:10:38.5:1.5.

### (2) Preparation of LNP with high nitrogen-to-phosphorus (N/P) ratio

The N/P molar ratio of nitrogen contained in the ionizable lipid of LNP prepared by the invention to the phosphorus contained in the encapsulated nucleic acid can be increased to 10:1-50:1. For the preparation of the LNP with added retinoid compound, the five-component lipid is formed by adding a certain proportion of ATRA, 9-CRA, 13-CRA or AM80, etc. on the basis of the four-component LNP, and each component is dissolved in the ethanol to form the organic phase. DNA (CpG-FAM) or mRNA (Luciferase mRNA) is dissolved in 0.05 M citrate buffer to form the aqueous phase with a nucleic acid concentration of 0.02 mg/mL-0.10 mg/mL. The LNP with high nitrogen phosphorus molar ratio is prepared by the microfluidic method or the ethanol dilution method, and then dialyzed in the 1 × PBS for more than 4 h.

### (3) Ratio and quantification of retinoid compounds added in five-component LNP

The addition ratio of the retinoid compound in the five-component LNP is based on the relative molar ratio to the cholesterol, and the molar ratio of the cholesterol to added retinoid compound is within a range of 1:2-50:1, preferably, 4:1-1:1.

Unless otherwise specified below, the five-component LNP includes the SM-102, the DSPC, the cholesterol, the DMG-PEG2000, and the ATRA/AM80 with a molar ratio of 36.1:7.2:27.8:1.1:27.8.

The content of the retinoid compound in the LNP is determined by high performance liquid chromatography (HPLC), including: A gradient dilution standard solution of ATRA or AM80 is prepared, the HPLC method is established, 100 µL of different concentrations of the standard solution is injected in turn, and the standard curve is plotted by using the ultraviolet absorption peak areas of different concentrations of ATRA or AM80. The five-component LNP is broken by ethanol with a volume more than 10 times the volume of the LNP, and the volume of each injection is controlled to be 100 µL; the peak area is substituted into the standard curve to obtain the content of ATRA or AM80 in the lipid solution.

### (4) Validation of the enhanced cellular uptake efficiency of the five-component LNP

The five-component LNP containing CpG-FAM is prepared using CpG-FAM as the encapsulated nucleic acid, and the prepared LNP is co-incubated with a dendritic cell DC2.4 cell line; the average fluorescence intensity of the cells is measured by flow cytometry to validate the cell entry efficiency of the LNP. It includes the following steps, the five-component LNP containing the retinoid compound is used as the experimental group, and the four-component LNP is used as the control group, and the same experimental operation is performed. After DC2.4 cells are affixed to the wall and co-incubated with the LNP encapsulated with the CpG-FAM for a period, the average fluorescence intensity of DC2.4 cells is detected by a FITC channel of the flow cytometer to determine LNP's cellular uptake efficiency.

### (5) Validation of five-component LNP in enhancing the mRNA expression in vitro

The mLuc-containing LNP is prepared using mLuc as the encapsulated nucleic acid; the prepared LNP is co-incubated with DC2.4 cells, and the mRNA expression efficiency of the LNP is verified by detecting the bioluminescence intensity with a microplate reader, including following steps. The five-component LNP containing the retinoid compound is used as an experimental group, and the four-component LNP is used as a control group, and the same experimental operation is performed. After DC2.4 cells are affixed to the wall and co-incubated with LNP encapsulated with the mLuc, the mRNA expression of the LNP is verified by detecting the bioluminescence intensity with the microplate reader using a firefly luciferase assay kit.

### (6) Validation of the generalizability of enhancing LNP mRNA expression by the retinoid compound

The mLuc is as the encapsulated nucleic acid, the nitrogen phosphorus molar ratio of nitrogen contained in the ionizable lipid to phosphorus contained in the encapsulated nucleic acid is 5.67:1, the ionizable lipid is 8-[(2-hydroxyethyl)(6-oxo-6-decyloxyhexyl)amino]octanoic acid(heptadecan-9-yl)ester(SM-102), [(4-hydroxybutyl) azetidinyl]bis(hexane-6,1-diyl)bis(2-hexylhexanoate)(ALC-0315), 4(N,N-dimethylamino)butanoic acid(dioleyl)methyl ester(DLin-MC3-DMA), 3,6-bis{4-[bis(2-hydroxydodecyl)amino]butyl}piperazine-2,5-dione(cKK-E12), 9-(4-(dimethylamino)butanoyloxy)heptadecanedioic acid di((Z)-nonly-2-en-1-yl) ester (L319), respectively, different LNPs containing the mLuc are prepared, and the prepared LNPs are co-incubated with DC2.4 cells, and bioluminescence intensity is detected using the microplate reader to verify the mRNA expression efficiency of the LNP. The above four-component LNPs containing different ionizable lipids are used as the control group, and the five-component LNPs containing the retinoid compound are used as the experimental group, and the same experimental operation is performed. After DC2.4 cells are affixed to the wall and co-incubated with the LNP encapsulated with the mLuc, the mRNA expression of the LNP is verified by detecting the bioluminescence intensity with the microplate reader using the firefly luciferase assay kit.

### (7) Validation of the five-component LNP for in vitro induction of T cell to express of gut-homing receptors

The five-component LNP containing mLuc is prepared using the mLuc as the encapsulated nucleic acid. T cells are sorted from mice spleen single cell suspensions using a mice T cell sorting kit. The sorted T cells are co-incubated with CD3/CD28 monoclonal antibody-coupled magnetic beads and the LNP. After 5 days of co-culture, the expression levels of C-C chemokine receptor 9(CCR9) and α4β7 integrin on the surface of T cells are detected by flow cytometry.

### (8) Validation of the five-component LNP in enhancing mRNA expression in animals.

The LNP containing mLuc is prepared using the mLuc as the encapsulated nucleic acid, and the prepared LNP is injected intramuscularly into C57BL/6 mice; the bioluminescence intensity is detected using a Small Animal In Vivo Imaging System(IVIS). It includes the following steps, the five-component LNP containing the retinoid compound is used as the experimental group, and the four-component LNP is used as the control group, and the same experimental operation is performed. 100 µL of LNP is injected into the muscle site of the hind leg of C57BL/6 female mice, and bioluminescence imaging is performed after a period of time, before imaging, mice are injected intraperitoneally with 200 µL of firefly luciferase substrate (D-luciferase potassium salt) at a concentration of 20 mg/mL; after 10 min of substrate injection, bioluminescence imaging is performed by IVIS to verify the in vivo mRNA expression efficiency of the LNP.

### (9) Validation of the five-component LNP simultaneously in inducing triple immune responses of humoral, cellular, and mucosal immunity

The LNP containing S-Omicron is prepared using mRNA encoding the spike protein of the Omicron strain of New Coronavirus (hereinafter referred to as S-Omicron) as the encapsulated nucleic acid; the prepared LNP solution is subcutaneously injected into C57BL/6 mice for immunization to verify the prophylactic immunization effect of LNP on an infectious disease model. it includes the following steps, the five-component LNP containing the retinoid compound is used as the experimental group, and the four-component LNP is used as the control group, and the same experimental operation is performed. The prepared LNP is injected subcutaneously into healthy C57BL/6 mice, the first injection is given at week 0, and the second injection is given at week 3 to enhance the immunity. At week 5, serum, feces, bronchoalveolar lavage fluid (BAL), lung tissue, and spleen are sampled from the mice, humoral, cellular, and mucosal immunity related indexes are detected by enzyme-linked immunosorbent assay (ELISA), enzyme-linked immune spot analysis (ELISpot), and the flow cytometry.

### (10) Anti-tumor effect of five-component LNP in an in situ colorectal tumor model

The LNP containing mOVA is prepared using mRNA encoding chicken ovalbumin (hereinafter referred to as mOVA) as the encapsulated nucleic acid. The prepared five-component LNP is injected intramuscularly into the muscle site of the hind leg of C57BL/6 mice with a homozygous tumor in situ in the cecum (MC38-Luc-OVA), and the anti-tumor effects of the LNP on the in situ colorectal tumor model are verified by IVIS and flow cytometric. MC38-Luc-OVA is a C57BL/6 mice MC38 colon cancer cell line capable of stably expressing firefly luciferase protein and chicken ovalbumin. It includes the following steps, the five-component LNP containing the retinoid compound is used as the experimental group and the four-component LNP is used as the control group, and the same experimental operation is performed. The prepared LNP is injected into the muscle site of the hind leg of the tumor-bearing C57BL/6 mice, the first injection is given on day 3 after in situ tumor implantation, and the second injection is given on day 8 after in situ tumor implantation to enhance immunity, tumor growth is monitored by the IVIS. Mice intestinal tumors are stripped on day 15, and the content of intra-tumor killer T cells is measured using flow cytometry.

The technical scheme of the invention is further explained in detail with specific embodiments in the following. It should be understood that the following embodiments illustrate and explain the invention only as examples, and should not be interpreted as limiting the scope of protection of the invention. All the technologies realized based on the above contents of the invention are covered within the scope of protection of the invention.

Unless otherwise stated, the raw materials and reagents used in the following embodiments are commercially available goods or can be prepared by known methods.

### Embodiment 1

Preparation of lipid solution: All lipids were pre-dissolved in ethanol and prepared as a stock solution for backup, the appropriate amount of DNA or mRNA was selected according to the experimental needs and the selected DNA or mRNA was diluted with 0.05 M citrate buffer to the required concentration, the dose of the used SM-102 was calculated on the basis of N/P (the nitrogen phosphorus molar ratio of nitrogen contained in the ionizable lipid to phosphorus contained in the encapsulated nucleic acid) of 5.67, and only the count of nitrogen atoms of the major lipid, i.e., SM-102, was counted when calculating the nitrogen phosphorus molar ratio, the amount of the remaining lipids was calculated based on a molar ratio of each lipid (SM-102:DSPC:Cholesterol:DMG-PEG-2000=50:10:38.5:1.5), and the retinoid compound (FIG. 1) was added according to a molar ratio of the retinoid compound to the cholesterol of 1:2. Ethanol was added to the lipid mixture so that the final volume ratio of the nucleic acid solution to the lipid solution is 3:1.

### Preparation of LNP

(1) Preparation of LNP by the ethanol dilution method: the appropriate volume of nucleic acid stock solution was taken, and CPG-FAM or mLuc stock solution was diluted to 0.05 mg/mL (aqueous phase) with 0.05 M citrate buffer to make the final volume of 90 µL, according to the volume ratio of the nucleic acid solution to the lipid solution of 3:1, 30 µL of lipid solution (organic phase) was prepared, the two phases were mixed and then the aqueous phase was aspirated into the organic phase, a liquid was mixed by rapidly aspirating the liquid with a pipette greater than 50 times, and then left to stand for 10 min, the prepared LNP was then dialyzed in a 1×PBS buffer solution with the volume of greater than 1000 times of the volume of the LNP for more than 4 h, and then placed in a refrigerator at 4°C for use.
(2) Preparation of LNP by the microfluidic method: the appropriate volume of the nucleic acid stock solution was taken, CPG-FAM or mLuc stock solution was diluted to 0.17 mg/mL (the aqueous phase) with 0.05 M citrate buffer to make the final volume of 300 µL, according to the volume ratio of the nucleic acid solution to the lipid solution of 3:1, a diploid volume (200 µL) of the lipid solution (organic phase) was prepared, and the two phases were mixed, the organic phase and the aqueous phase were aspirated using a 1 mL insulin needle, and the organic phase and the aqueous phase were injected into a microfluidic chip for mixing at a flow rate of 0.3 mL/min and 0.9 mL/min, respectively, the liquid was stopped collecting after the aqueous phase was empty in the insulin needle, and the prepared LNP was dialyzed in a 1×PBS buffer solution with a volume greater than 1000 times of the volume of the LNP for more than 4 h, and then placed in the refrigerator at 4°C for use.

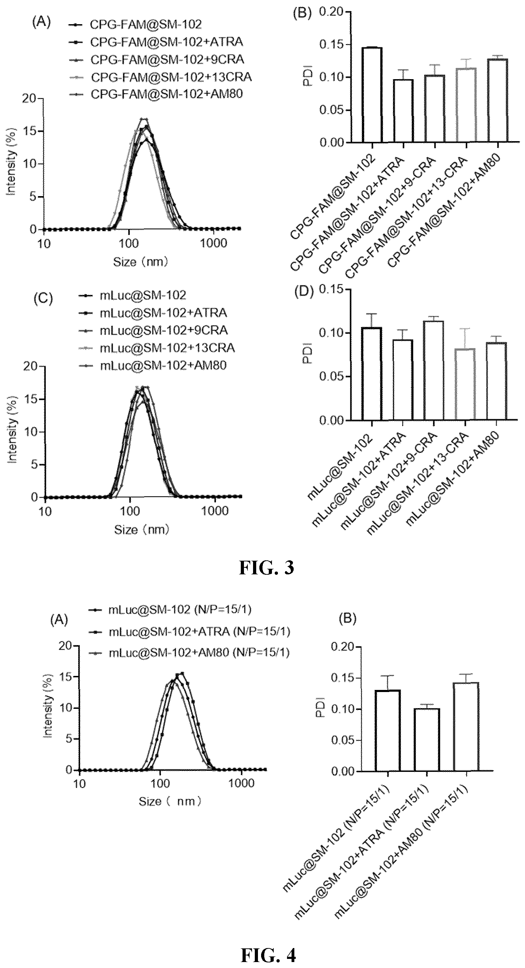

FIG. 3 shows the normal distribution curves of hydrated particle sizes of prepared nanoparticles characterized by Dynamic Light Scattering (DLS), hydrated particle sizes and PDIs of CpG-FAM@SM-102, CpG-FAM@SM-102+ATRA, CpG-FAM@SM-102+9-CRA, CpG-FAM@SM-102+13-CRA, CpG-FAM@SM-102+Am80 LNP and mLuc@SM-102, mLuc@SM-102+ATRA, mLuc@SM-102+9-CRA, mLuc@SM-102+13-CRA, and mLuc@SM-102+Am80 LNP are 159.5 nm, 0.146; 151.0 nm, 0.097; 157.6 nm, 0.104; 130.1 nm, 0.114; 133.5 nm, 0.127; 123.6 nm, 0.107; 132.3 nm, 0.093; 136.6 nm, 0.114; 124.6 nm, 0.082; and 147.2 nm, 0.089.

### Embodiment 2

Lipid solution and LNP were prepared in the same manner as stated in Embodiment 1. The amount of lipid was calculated based on N/P (the nitrogen phosphorus molar ratio of nitrogen contained in the ionizable lipid to phosphorus contained in the encapsulated nucleic acid) of 15/1. Only the count of nitrogen atoms was counted in the major lipid, i.e., SM-102 when calculating the nitrogen and phosphorus molar ratio. DNA or mRNA was diluted to 0.1 mg/mL with 0.05 M citrate buffer.

FIG. 4 shows normal distribution curves of the hydrated particle size of prepared nanoparticles characterized by DLS, hydrated particle sizes and PDIs of mLuc@SM-102 LNP, mLuc@SM-102+ATRA LNP, and mLuc@SM-102+AM80 LNP are 158.8 nm, 0.131; 173.6 nm, 0.102; and 141.7 nm, 0.143.

### Embodiment 3

Chromatographic conditions: Sunfire C18 column (150 mm×4.6 mm, 5 µm), column temperature (25°C), mobile phase A (acetonitrile), mobile phase B (0.1 M triethylamine acetate buffer solution with a pH of 7.0), flow rate of 1.0 mL/min, and injection volume of 100 µL each time.

The standard solutions of AM80 with different concentrations were prepared with ethanol, and HPLC injection and gradient elution were performed, the proportion of the mobile phase A increased from 0% to 100% from 0 min to 15 min, with a detection wavelength of 275 nm, and the peak time of AM80 is about 10.5 min, the peak area-concentration standard curve of AM80 was obtained by integrating the absorption peak area: y=222918*x -161172 (R²=0.9992, 0<x<100 µg/mL), where x is the mass concentration of AM80 solution, and y is the corresponding AM80 absorption peak area. After diluting LNP with the ethanol, HPLC injection was performed, the AM80 content in the LNP solution was quantified according to the absorption peak area at 275 nm, and the encapsulation efficiency of AM80 is 45%-65%.

The standard solutions of different concentrations of ATRA were prepared with the ethanol, and HPLC injection and isocratic elution were performed, the ratio of mobile phase A to mobile phase B was 95:5, and the detection wavelength was 350 nm, the peak time of ATRA was about 5 min, and the peak area-concentration standard curve of ATRA was obtained by integrating the absorption peak area: y=436478*x+257220 (R²=0.9996, 0<x<200 µg/mL), where x is the mass concentration of ATRA solution, and y is the corresponding ATRA absorption peak area. After dilution of LNP with the ethanol, HPLC injection was performed, the content of ATRA in the LNP solution was quantified based on the absorption peak area at 350 nm, and the encapsulation efficiency of ATRA is 40%-70%.

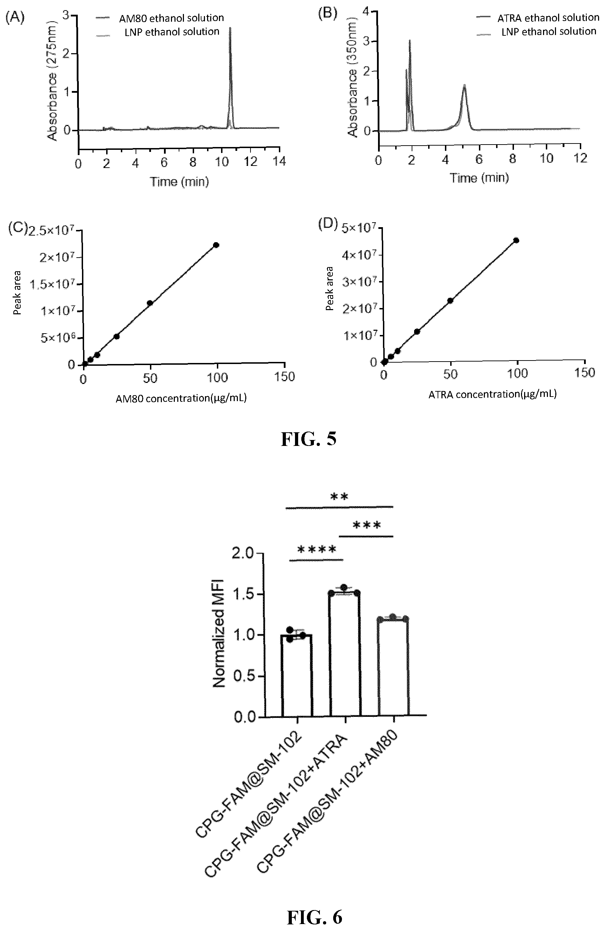

The HPLC spectra and the corresponding peak area-concentration standard curves of AM80 and ATRA are shown in (A) (B) (C) (D) of FIG. 5, respectively.

### Embodiment 4

The LNP containing CpG-FAM was prepared according to the manner stated in Embodiment 1, and the five-component LNP containing the retinoid compound was used as the experimental group (CpG-FAM@SM-102+ATRA, CpG-FAM@SM-102+AM80 LNP), the four-component LNP was used as the control group (CpG-FAM@SM-102), and the same experimental operation was performed to evaluate the efficiency of the lipid nanoparticle entering cells. On the day before the experiment, DC2.4 cells were spread on the plate, and about 50,000 cells were added into each well of the 48-well cell culture plate, when the cell confluence was 70-80%, LNP was added and mixed with slight shaking, then incubated in a saturated humidity incubator with 5% CO₂ at 37°C for 2-4 h, the cells were digested by trypsin and separated to obtain a single-cell suspension, and the average fluorescence intensity of the cells was detected using flow cytometry to validate the efficiency of the LNP uptake by DC2.4 cells. As shown in FIG. 6, the efficiency of the five-component LNP containing the retinoid compound uptake by DC2.4 cells is significantly higher than that of the four-component LNP.

### Embodiment 5

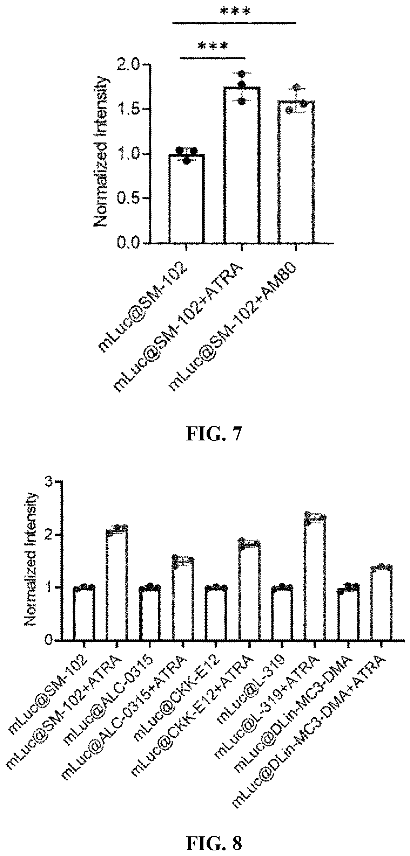

The LNP containing mLuc was prepared according to the manner stated in the Embodiment 2. The four-component LNP was used as the control group (mLuc@SM-102), and the five-component LNP with added retinoid compound was used as the experimental group (mLuc@SM-102+ATRA, mLuc@SM-102+AM80), and the prepared LNP was used for transfection of cells to test the efficiency of the LNP in delivering mRNA. On the day before transfection, DC2.4 cells were plated in 96-well cell culture plates, and about 10,000 cells were added to each well, LNP was added and mixed with slight shaking when the cell confluence was 70%-80%, and then cultured in a saturated humidity incubator with 5% CO₂ at 37°C for 24 h. The culture plate was taken out, after the temperature of the culture plate equilibrated to room temperature, one-half of the volume of culture medium of each well was removed and 100 µL of fluorescein substrate was added to each well, and the culture plate was placed on the shaker for shaking for 5 min to make the cells fully lysed, then the lysate was transferred to a white 96-well plate for detecting the intensity of bioluminescence signal with the microplate reader, the result was shown in FIG. 7. As shown in FIG. 7, the bioluminescence intensity of the five-component LNP containing the retinoid compound is significantly stronger than that of the four-component LNP, indicating that the five-component LNP increases the efficiency of mLuc expression in the cells.

### Embodiment 6

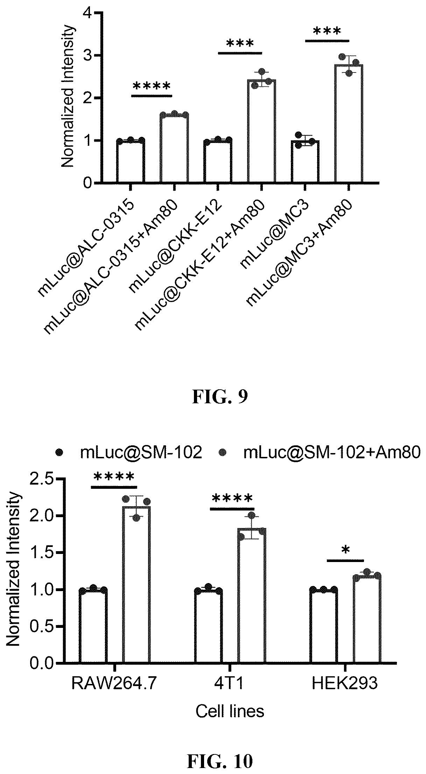

The LNP containing mLuc was prepared according to the manner of Embodiment 2, and the five-component LNP containing the retinoid compound was used as the experimental group (mLuc@SM-102+ATRA, mLuc@ALC-0315+ATRA, mLuc@CKK-E12+ATRA, mLuc@L319+ATRA, mLuc@MC3+ATRA, mLuc@ALC-0315+Am80, mLuc@CKK-E12+Am80, mLuc@MC3+Am80), the four-component LNP was used as the control group (mLuc@SM-102, mLuc@ALC-0315, mLuc@CKK-E12, mLuc@L319, mLuc@MC3), and the same experimental operation was performed. The methods of detecting the efficiency of LNP in delivering mRNA, transfecting cells, and the intensity of bioluminescent signal are consistent with Embodiment 5. As shown in FIG. 8 and FIG. 9, there is a significant increase in the mRNA expression efficiency of five-component LNP containing ATRA or Am80 in each group as compared to the four-component LNP, indicating that the five-component LNP containing the retinoid compound is generalizable in improving mRNA expression.

### Embodiment 7

The LNP containing mLuc was prepared according to the manner of the Embodiment 2. The five-component LNP containing the retinoid compound was used as the experimental group (mLuc@SM-102+Am80), the four-component LNP was used as the control group (mLuc@SM-102), and the same experimental operation was performed. The methods of detecting the efficiency of LNP in delivering mRNA, transfecting cells, and the intensity of bioluminescent signal were consistent with Embodiment 5. The cells were replaced with mice macrophage RAW264.7, mice tumor cell 4T1, or human embryonic kidney cell HEK293. As shown in FIG. 10, the mRNA expression efficiency of each set of five-component LNP containing Am80 is significantly increased compared with that of the four-component LNP, indicating that the five-component LNP containing the retinoid compound can improve mRNA expression in different cell types.

### Embodiment 8

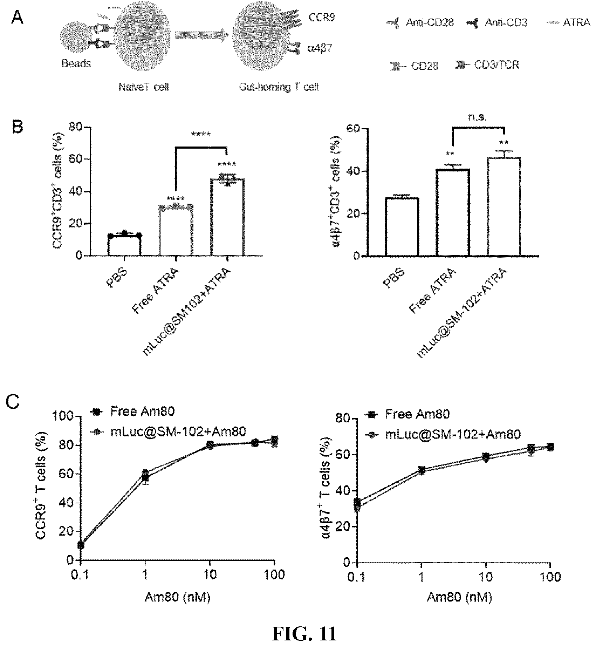

The LNP containing mLuc was prepared according to the manner of Embodiment 1. The five-component LNP (mLuc@SM-102+ATRA, mLuc@SM-102+Am80) containing the retinoid compound was used as the experimental group, the four-component LNP was used as the control group, and the same experimental operations was performed. Spleens of C57BL/6 mice were extracted and immersed in ice-cold sterile PBS buffer solution, the spleens were clamped with sterile forceps and placed on 200-mesh nylon mesh, which was placed in a petri dish containing 5 mL of ice-cold PBS buffer solution, and the mice spleen was ground with a 5 mL syringe plunger, the cell suspension was collected and centrifuged, the supernatant was discarded, and the lysed erythrocytes were washed twice with PBS buffer solution. The T cells were sorted according to the instructions of the mice T cell isolation kit, The sorted T cells were cultured using a 96-well cell culture plate with about 100,000 cells per well, and CD3/CD28 monoclonal antibody-coupled magnetic beads and LNP were added for co-culturing with the T cells. After 5 days of culture, the cells were aspirated into 1.5 mL EP tubes, washed twice with PBS buffer solution, and then stained with antibodies, and the ratios of CCR9⁺T cells and α4β7+T cells were detected using flow cytometry. As shown in FIG. 11, the five-component LNP containing ATRA or Am80 is still effective in inducing T cells to expressgut-homing receptors CCR9 and α4β7 compared to free ATRA or free Am80.

### Embodiment 9

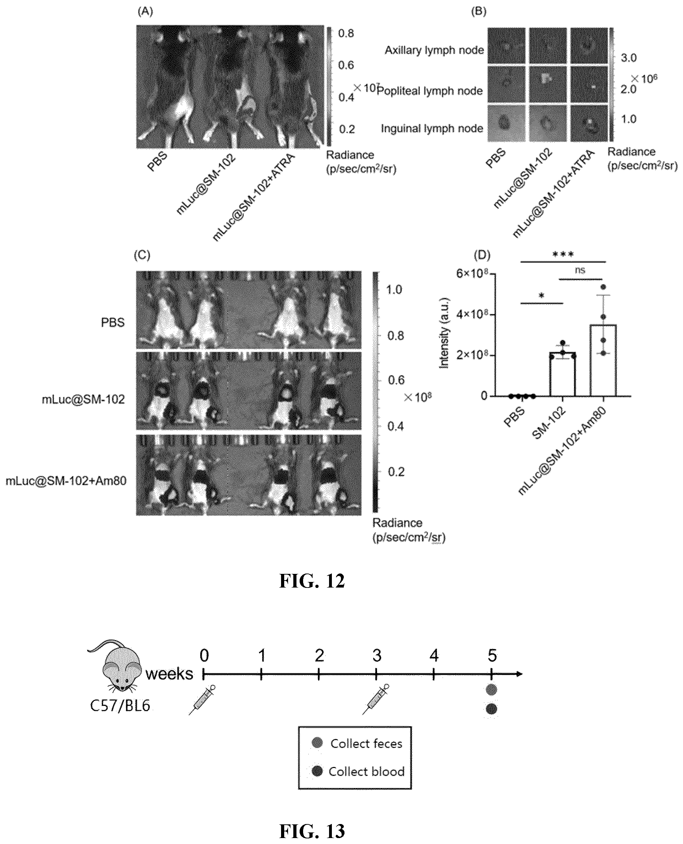

The LNP containing mLuc was prepared according to the manner of Embodiment 2. The five-component LNP containing the retinoid compound was used as the experimental group (mLuc@SM-102+ATRA, mLuc@SM-102+AM80), the four-component LNP (mLuc@SM-102) and PBS solution were used as the control groups, and the same experimental operation was performed. 100 µL of LNP solution or PBS solution was injected into the muscle site of the hind leg of female C57BL/6 mice, with an administered dose of 5 µg mRNA. After 6 hours, 160 µL of 20 mg/mL luciferase substrate was injected intraperitoneally, and the in vivo bioluminescent signal was detected by the small animal IVIS after 10 min. After in vivo imaging, the mice were euthanized and the lymph nodes were extracted for in vitro bioluminescence imaging. As shown in FIG. 12, the in vivo and ex vivo imaging results demonstrate that the efficiency of mRNA expression in vivo of the five-component LNP containing the retinoid compound is significantly higher than that of the four-component LNP.

### Embodiment 10

The LNP containing spike protein mRNA (S-omicron) of the omicron strain of the new coronavirus was prepared according to the manner of Embodiment 2. The five-component LNP containing the retinoid compound was used as the experimental group (S@SM-102+AM80), the four-component LNP (S@SM-102) and the PBS solution were used as the control groups, and the same experimental operations were performed. Healthy female C57BL/6 mice at 6-8 weeks were used for the experiment, with 5 mice in each experimental group. Each mouse was immunized subcutaneously at week 0 by administering a dose of LNP solution containing 20 µg of S-omicron, with 200 µg of AM80 in five-component LNP. The second booster immunization was carried out at week 3 with the same operation as the first operation, and the experimental procedure was shown in FIG. 13. Blood sampling from the orbital vein of mice was performed at week 2 after booster immunization, and serum was collected from each mouse, and the serum was assayed for the titer of the specific binding antibody against omicron spike protein, characterizing the efficiency of activation of humoral immunity.

Single cell suspensions were prepared by grinding the spleens of mice, and the splenocytes were cultured in 96-well plates with 1 million cells per well and stimulated with omicron peptide library (3 µg/mL), and protein transport inhibitors were added after 8 h of culture. After the culture was continued for 4 h, the omicron specific T cells (CD3⁺CD4⁺IFN-γ⁺, CD3⁺CD8⁺IFN-γ⁺) were measured by flow cytometry. Splenocytes were cultured by using 96-well plates from the ELISPOT kit with 300,000 cells per well, stimulated with omicron spike protein (8 µg/mL), and the number of omicron-specific cells was detected after 24 h of culture according to the instructions of the kit, characterizing the efficiency of activation of cellular immunity.

The bronchoalveolar lavage (BAL) and feces of mice were taken, and the titers of the omicron S protein-specific IgA antibody in BAL and feces were measured by ELISA kits, and lung monocyte suspensions were prepared by taking lung tissues from mice, and the content of tissue-resident T cells (CD69⁺CD103⁺CD8⁺) was measured in the lungs of mice by flow cytometry, which was used to characterize the efficiency of activation of mucosal immunity.

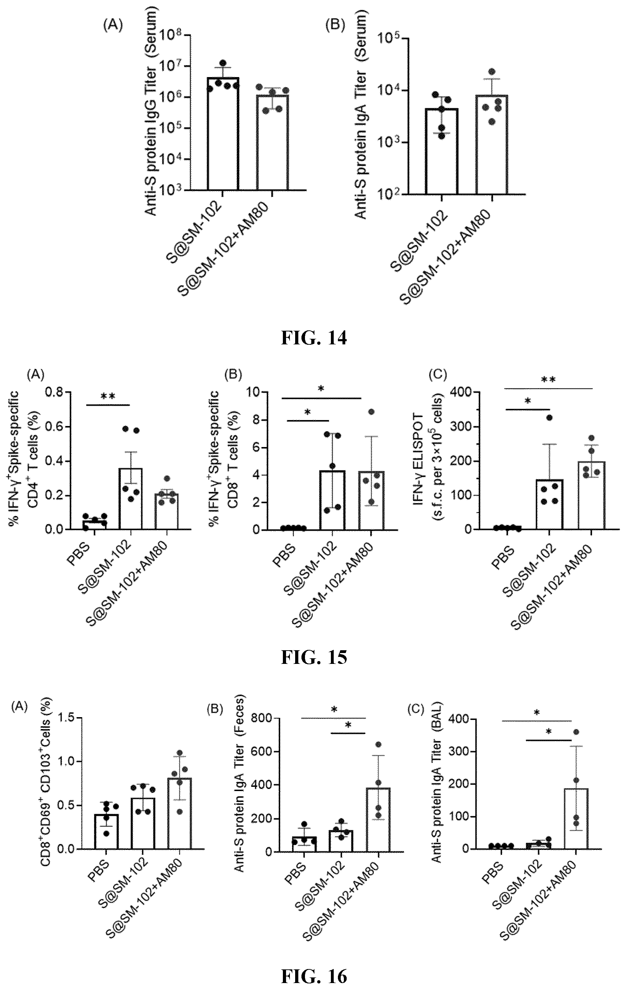

As shown in FIG. 14, the results of serum antigen-specific IgG and IgA antibody titers indicates that compared to the PBS group, both S@SM-102 and S@SM-102+AM80 produce high levels of serum antigen-specific IgG and IgA antibody titers, indicating that both efficiently activate humoral immune response. As shown in FIG. 15, the results of flow cytometry and ELISPOT indicate that compared to the PBS group, the number of omicron-specific T cells in the spleens of mice inoculated with S@SM-102 and S@SM-102+AM80 and the number of splenocytes secreting type I interferon after antigen stimulation significantly increase, indicating that both efficiently activate the cellular immune response. As shown in FIG. 16, the flow cytometric results of lung tissues indicate that the content of lung tissue-resident T cells increases in the S@SM-102+AM80 group compared with the PBS group and the S@SM-102 group. In BAL and feces, the omicron S protein specific IgA antibody was barely detectable in the PBS and S@SM-102 groups, while the IgA antibody titers significantly increase in the S@SM-102+AM80 group, indicating that the four-component LNP fails to activate the mucosal immune response, and the five-component LNP containing the retinoid compound efficiently activates the mucosal immune response.

Technicians in this field can understand that by selecting encapsulated nucleic acids, a variety of vaccines can be obtained: influenza vaccine, HIV vaccine, viral pneumonia (SARS, SARS-CoV-2) vaccine, tuberculosis vaccine, respiratory syncytial virus (RSV) vaccine, norovirus vaccine, rotavirus vaccine, etc.

### Embodiment 11

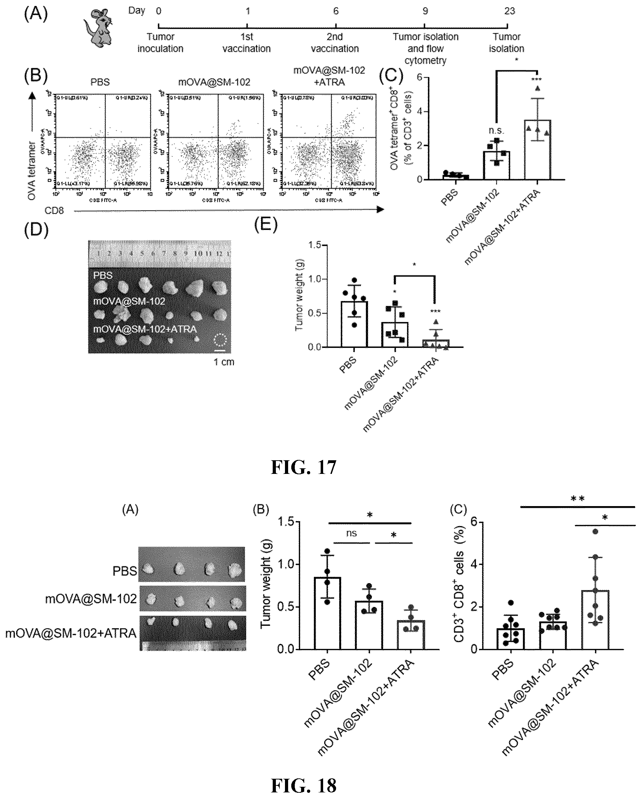

The LNP containing chicken ovalbumin mRNA (mOVA) was prepared according to the manner of Embodiment 2, the five-component LNP containing the retinoid compound (mOVA@SM-102+ATRA) was used as the experimental group, the four-component LNP (mOVA@SM-102) and PBS solution was used as the control group, and the same experimental operation was performed. Subcutaneous inoculation of the MC38-Luc-OVA cell line was performed, when the volume of the subcutaneous tumors reached about 1000 mm³, the subcutaneous tumors were peeled off and sheared into lumps with a length and width about 2 mm, and the patches were planted in the cecum of 4-6 week-old female C57BL/6 mice to construct an in situ colorectal tumor model. On day 1 after tumor implantation, the prepared LNP or PBS solution was injected into the muscle sites of the hind legs of the tumor-bearing C57BL/6 mice at a dose of 10 µg of mRNA, with 70 µg of ATRA in the five-component LNP (FIG. 17A). The second enhancement injection was performed on day 6. Mice were intraperitoneally injected with 160 µL of 20 mg/mL of D-luciferin potassium salt at different time points after tumor inoculation, respectively, and the tumor growth was monitored by detecting the bioluminescence intensity of the tumors using the Small Animal IVIS. On day 9, 4 mice from each group were euthanized, the intestinal tumors were stripped, a single-cell suspension of the tumors was prepared, the number of CD3⁺CD8⁺ T cells in the tumors was detected by flow cytometry, and the tumor growth of the remaining mice continued to be monitored using the Small Animal IVIS.

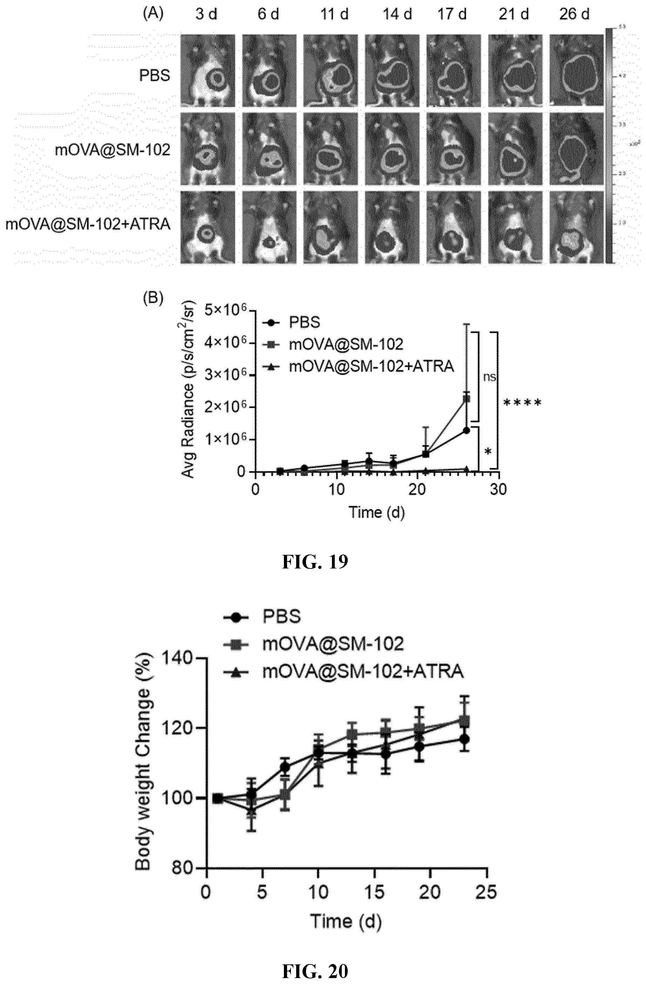

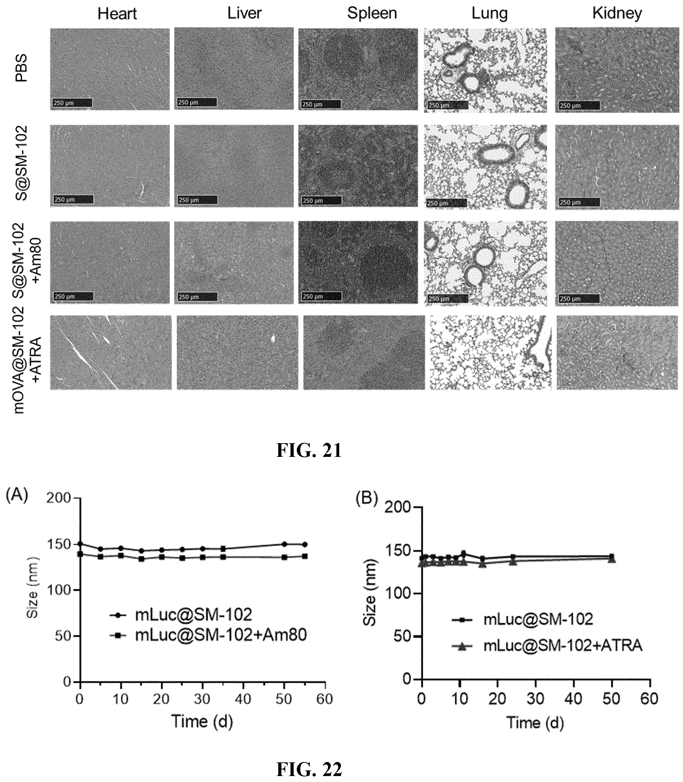

As shown in FIG. 17 and FIG. 18, the flow cytometry results indicate that compared to PBS and mOVA@SM-102, the content of intra-tumor killer T cells in mice vaccinated with the mOVA@SM-102+ATRA vaccine significantly increases, indicating that the five-component LNP containing the retinoid compound can induce the homing of subcutaneously activated T cells in intestinal tumors, which is expected to improve the tumor suppression effect. As shown in FIG. 17-FIG. 19, the results of tumor images and growth curves indicate that compared with PBS and mOVA@SM-102, the five-component LNP containing the retinoid compound has a significant inhibitory effect on in situ colorectal tumors in mice. As shown in FIG. 20, there is no significant change in body weight of mice after inoculation with mOVA@SM-102+ATRA and mOVA@SM-102. As shown in FIG. 21, pathologic sections of major organs show that all experimental groups do not cause damage to major organs, demonstrating good biocompatibility.

### Embodiment 12

The LNP containing mLuc was prepared according to the manner of the Embodiment 2. The five-component LNP containing the retinoid compound (mLuc@SM-102+Am80, mLuc@SM-102+ATRA) was used as the experimental group, the four-component LNP (mLuc@SM-102) was used as the control group, and the same experimental operation was performed. The LNP solution was stored at 4°C and the LNP samples were taken out at different time points to characterize the particle size using a dynamic light scatterometer. As shown in FIG. 22, the five-component LNP is stable while stored at 4°C.

Above, the embodiments of the invention are described. However, the invention is not limited to the above embodiments. Any modification, equivalent replacement, improvement, etc. made within the spirit and principles of the invention shall be included within the scope of protection of the invention.

## Claims

1. A lipid nanoparticle (LNP), the lipid nanoparticle comprises a carrier and an encapsulated nucleic acid, the carrier comprises an ionizable lipid, a helper phospholipid, a PEGylated lipid, cholesterol and derivatives thereof, and a retinoid compound; and the nucleic acid is at least one of mRNA, circRNA, siRNA, microRNA, antisense nucleic acid, and plasmid.

2. The lipid nanoparticle according to claim 1, wherein the ionizable lipid accounts for 20 mol %-50 mol % of total lipids in the LNP;
and/or, the ionizable lipid is selected from at least one of the following compounds, comprising but not limited to: 8-[(2-hydroxyethyl)(6-oxo-6-decyloxyhexyl)amino]octanoic acid(heptadecan-9-yl)ester(SM-102), [(4-hydroxybutyl)azetidinyl]bis(hexane-6,1-diyl)bis(2-hexyldecanoate)(ALC-0315), 4-(N,N-dimethylamino)butanoic acid(dioleyl)methyl ester(DLin-MC3-DMA), 3,6-bis{4-[bis(2-hydroxydodecyl)amino]butyl}piperazine-2,5-dione(cKK-E12), 9-(4-(dimethylamino)butyryloxy)heptadecanedioic acid di((Z)-nonly-2-en-1-yl)ester(L319), N2,2-dimethylene-4-(dimethylamino)ethyl[1,3]-dioxolane(DLin-KC2-DMA), 8-[(2-hydroxyethyl)(8-nonyloxy-8-oxooctyl)amino]octanoic acid(heptadecan-9-yl)ester (Lipid5), 1,1'-[(2-{4-[2-({2-[Bis(2-hydroxydodecyl)amino]ethyl}(2-hydroxydodecyl)amino)ethyl ]piperazin-1-yl}ethyl)azabicyclo[bis(dodecane)-2-alcohol)(C12-200), (2,3-dioleoyl-propyl)trimethylammonium chloride(DOTAP), dimethyldioctadecylammonium bromide(DDAB) and tetrakis(8-methylnonyl)3,3',3",3"'-{[(methylazanediyl)bis(propane-3,1diyl)]bis(azatriyl) }tetrapropionate(306Oi10);
and/or, the helper phospholipid accounts for 2 mol %-10 mol % of the total lipids in the LNP;
and/or, the helper phospholipid is selected from at least one of the following compounds, comprising but not limited to: 1,2-distearoyl-sn-glycero-3-phosphatidylcholine (DSPC), 1,2-dioleoyl-sn-glycero-3-phosphatidylcholine (DOPC), 1,2-dipalmitoyl-sn-glycero-3-phosphatidylcholine (DPPC), 2-oleoyl-1- palmitoyl acyl-sn-glycero-3-phosphatidylcholine (POPC), 1,2-dioleoyl-sn-glycero-3-phosphatidylethanolamine (DOPE), 2-oleoyl-1-palmitoyl-sn-glycero-3-phosphatidylethanolamine (POPE), 1,2-distearoyl-sn-glycero-3-phosphatidylethanolamine (DSPE), and 1,2-dipalmitoyl-sn-glycero-3-phosphatidylethanolamine (DPPE);
and/or, the cholesterol and derivative thereof account for 10 mol %-40 mol % of the total lipids in the LNP;
and/or, the cholesterol and derivative thereof are selected from at least one of the following compounds, comprising but not limited to: β-sitosterol, cholestanol, cholestanone, cholesterol, cholestenone, 7β-hydroxycholesterol, and 7α-hydroxycholesterol;
and/or, the PEGylated lipid accounts for 0.3 mol % -30 mol % of the total lipids in the LNP;
and/or, the PEGylated lipid is selected from at least one of the following compounds, comprising but not limited to: 1,2-dimyristoyl-rac-glycero-3-methoxypolyethylene glycol (DMG-PEG), 1,2-distearoyl-rac-glycero-3-methoxypolyethylene glycol (DSG-PEG), 1,2-dipalmitoyl-rac-glycero-3-methoxy polyethylene glycol (DPG-PEG), and 1,2-distearoyl-sn-glycero-3-phosphatidylethanolamine-methoxypolyethylene glycol (DSPE-PEG);
and/or, the retinoid compound accounts for 1 mol %-35 mol % of the total lipids in the LNP;
and/or, the retinoid compound is at least one of retinoic acid derivatives and synthetic retinoid compounds, for example, the retinoic acid derivatives comprise, but not limited to, at least one of all-trans-retinoic acid (ATRA), 9-cis-retinoic acid (9-cis-RA, 9-CRA), 13-cis-retinoic acid (13-cis-RA, 13-CRA), and the synthetic retinoid compounds comprise, but not limited to, retinoic acid receptor agonist;
the 'total lipids' refer to a sum of the ionizable lipid, the helper phospholipid, the cholesterol and the derivatives thereof, the PEGylated lipid, and the retinoid compound.

3. The lipid nanoparticle according to claim 1 or 2, wherein a molar ratio of the cholesterol and the derivatives thereof to the retinoid compound is within a range of 1:2-50: 1;
and/or, an encapsulation efficiency of the nucleic acid is 30-99 %;
and/or, an encapsulation efficiency of the retinoid compound is 30-99 %;
and/or, a molar ratio of nitrogen element of the ionizable lipid to phosphorus element of the nucleic acid is about (1-50):1;
and/or, a hydrated particle size of the LNP is within a range of 100 nm-200 nm.

4. A preparation method of the LNP according to any one of claims 1-3, wherein the preparation method comprises obtaining the LNP based on an aqueous phase containing the nucleic acid and a lipid organic phase by an ethanol dilution method or a microfluidic method.

5. The preparation method according to claim 4, wherein the ethanol dilution method comprises the following steps: aspirating the aqueous phase containing the nucleic acid into the lipid organic phase, mixing a liquid by rapidly aspirating the liquid with a pipette greater than 50 times, standing after mixing to obtain the LNP;
preferably, the ethanol dilution method further comprises dialyzing the product in a 1×PBS buffer solution with a volume of greater than 1000 times of a volume of the product for more than 4 h.

6. The preparation method according to claim 4, wherein the microfluidic method comprises the following steps: aspirating the lipid organic phase and the aqueous phase containing the nucleic acid with a syringe, respectively, and injecting the lipid organic phase and the aqueous phase into a microfluidic chip at a flow rate of 1:3 for mixing, stopping collecting the liquid after the aqueous phase is empty in the syringe to obtain the LNP;
preferably, the microfluidic method further comprises dialyzing the collected mixture in a 1×PBS buffer solution with a volume of 1000 times of a volume of the mixture for more than 4 h.

7. An application of the LNP according to any one of claims 1-2 in a preparation of a biological product.

8. The application according to claim 7, wherein biological product is an injectable biological product;
preferably, the biological product is a vaccine, preferably, an mRNA vaccine;
preferably, the biological product is an influenza vaccine, a human immunodeficiency virus (HIV) vaccine, a viral pneumonia (SARS, SARS-CoV-2, etc.) vaccine, a tuberculosis vaccine, a norovirus vaccine, a rotavirus (RV) vaccine, a respiratory syncytial virus (RSV) vaccine, an enterovirus (e.g., EV71) vaccine, an intestinal mucosal-related tumor vaccine (e.g., colorectal cancer mRNA vaccine), or a lung cancer vaccine, etc.

9. The application according to claim 7 or 8, the biological product is used to prevent and/or treat mucosal transmission infectious diseases and mucosal-associated tumors; for example, the infectious disease is selected from influenza, novel coronavirus pneumonia (SARS-CoV-2), atypical pneumonia (SARS), tuberculosis, bronchitis and pneumonia due to RSV infection, AIDS, hand-foot-and-mouth disease due to enteroviral infections, diseases caused by rotaviruses, and diseases caused by noroviruses, for example, the mucosa-associated tumors can be colorectal tumors, lung cancer, etc.

10. A biological product, the biological product is the biological product according to claim 8 or 9.
